Europäisches Patentamt

European Patent Office

Office européen des brevets

(19)

(11) Publication number: **0 154 686 B1**

# EUROPEAN PATENT SPECIFICATION

(12)

(45) Date of publication of patent specification: **09.10.91**

(21) Application number: **84114260.7**

(22) Date of filing: **18.02.83**

(51) Int. Cl.5: **B01L 3/00**, G01N 33/53, G01N 33/545

(60) Publication number of the earlier application in accordance with Art.76 EPC: **0 087 899**

(54) **Carrier plate and multi-well screening assembly for immunoassay procedures.**

(30) Priority: **03.03.82 US 354554**

(43) Date of publication of application:
**18.09.85 Bulletin 85/38**

(45) Publication of the grant of the patent:
**09.10.91 Bulletin 91/41**

(84) Designated Contracting States:
**BE DE FR GB IT NL SE**

(56) References cited:
**EP-A- 0 040 943     EP-A- 0 042 755**
**FR-A- 2 277 563     FR-A- 2 362 397**
**FR-A- 2 369 557     US-A- 4 111 754**
**US-A- 4 200 613**

(73) Proprietor: **Becton, Dickinson and Company**
**Mack Centre Drive P.O. Box 2224**
**Paramus New Jersey 07652-1149(US)**

(72) Inventor: **Sapatino, Bruno V.**
**1401 S. Port Dr.**
**Oxnard, CA 93030(US)**
Inventor: **Johnson, Luther R.**
**878 Capitan Street**
**Thousand Oaks, CA 91320(US)**

(74) Representative: **Ruffles, Graham Keith et al**
**MARKS & CLERK 57-60 Lincoln's Inn Fields**
**London WC2A 3LS(GB)**

## Description

The present invention relates to a multi-well screening system useful in immunoassay procedures, and more particularly, concerns such a screening assembly suitable for the screening of hydbrid cell cultures such as hybridomas. The present invention also relates to a carrier plate useful in immunoassay procedures and having elements thereof adapted to absorb immunogenic substances thereto.

Monoclonal antibodies are being investigated by researchers for a variety of uses, including the treatment of cancer. It is now well known that monoclonal antibodies can be produced in large quantities from hybridomas. A hybridoma is a hybrid cell which has been produced by joining together a cancer cell with an antibody-making cell. Inasmuch as these hybridomas can produce specific antibodies in large quantities, it is desirable to be able to detect and quantify the presence of the specific hybridoma under investigation.

The search for a certain hybridoma can be very tedious and time-consuming. In the research laboratory, the quest for a certain antibody may start with the injection of an antigen, against which the certain antibody will form, into a mouse. Following removal of the mouse's spleen, the spleen cells are fused with cancer cells. It is to be appreciated that after this process of fusing cancer cells and spleen cells, the researcher must be able to detect which of these fused cells are actually producing the desired antibodies. Only a very small percentage of the cells which the researcher started with will actually fuse together to form a hybrid. This search for fused cells usually includes the distribution of the combined spleen cells and cancer cells into various compartments in order to make the proper identification. Eventually, the researcher attempts to isolate not only the hybridomas, but also to isolate those hybridomas which are producing the sought for antibody. Once again, the researcher may rely upon various compartmentalized trays or tubes in order to complete this isolation.

In the detection and quantification of the various proteins, such as monoclonal antibodies produced by hybridomas, the researcher may rely upon one or more assays. For example, he may use the enzyme-linked immunosorbent assay (ELISA) or the radioimmunoassay (RIA). These immunoassays are very well known to researchers in this field. Both of these assays may utilize test equipment including multiple compartments or wells so that processing of each may be performed simultaneously on a microtiter basis. For example, Schlieicher and Schuell, Inc, Keene, New Hampshire 03431, USA, utilize a 96 place microsample filtration manifold which has direct application in the DNA/RNA hybridization research field. Also, Klebe, Robert J, et al, in "Replica Plating of Cultured Mammalian Cells," Somatic Cell Genetics, Vol 7, No 3, 1981, pp 271-180, describe a device which permits the replica plating of lymphocytes as well as substrate adherent fibroblasts. In this study, the authors relied on ELISA assays with 96 well microtiter plates.

Beads have been utilized in these assay procedures in order to link antigen or antibody thereto in the detection of the sought for biological substance. For example, Litton Bionetics Laboratory Products, Kensington, Maryland, USA, have described the use of a "BIO-BEAD" which is used for the rapid screening of serum specimens for the presence of detectable antibodies to the TORCH group of antigens. These beads include a magnetic component for use in the Litton Bionetics' system. Micro-Media Systems, Inc, Potomac, Maryland, USA, have disclosed an MIC and bacterial identification device which includes a tray with a multiplicity of wells and a cover with downwardly depending pins which fit in alignment in the wells. These pins are adapted to permit protein (antigen or antibody) to become adsorbed to the surface thereof. It has been found, however, that elongate pins may cause a capilliary action when dipped into fluid in the wells. As a result, accuracy of fluid transferred to the pin may be compromised. Furthermore, fluid sometimes drips from these pins after the cover is removed from the tray with the wells. This may result in cross-contamination of the fluid into adjacent wells.

In US Patent 4200613 there is described a carrier for use with a test tray having reaction chambers. The carrier has removable grippers for gripping antigen- or antibody-coated beads.

While the above mentioned devices may be known and used, improvements are being sought in the multiple-well approach to screening of biological substances, such as hybridomas. It is to such improvements that the present invention is directed.

The present invention provides a carrier plate useful in immunoassay procedures and comprising:
a support body, and
a plurality of posts, extending from a face of the body with each post having an enlarged removable portion at its distal end adapted to bind an immunogenic substance to its surface, each post having a reduced neck to render it frangible for removal of the enlarged portion from the plate.

The present invention also provides a multi-well screening assembly which comprises a tray having a plurality of individualized wells therein and a carrier plate of the invention for positioning on the tray. Each carrier post aligns with one of the wells in order that the enlarged portion at its distal end can depend into the aligned well.

In a preferred embodiment of the aforementioned aspects of the invention, the tray includes 96 wells arranged in orthogonal columns and rows, with the carrier plate including a corresponding number of posts for alignment with the wells. Each post includes a substantially spherical bead removably connected to the distal end of the post. This bead is particularly adapted to absorb proteins on its surface and, to this end, is preferably made of plastic, with the plastic of choice being styrene acrylonitrile.

A further aspect of the present invention includes a multi-well screening assembly together with a carrier plate which comprises:

a generally planar support body; and

a plurality of separate, individualized pockets in the body intended for receipt of individual enlarged portions after removal from the carrier plate by rupture of the respective frangible neck;

each pocket including an upper opening to allow deposit of the respective removable portion to the respective pocket and to allow removal of the removable portion from the pocket, and each pocket further including a lower opening which is smaller than the removable portion deposited in the pocket and which allows fluid to enter in to the pocket while the removable portion is in the pocket.

In accordance with the present invention, simultaneous processing of all wells of a microtiter for biological substance screening purposes can be achieved. By employing an immunoassay, such as ELISA or RIA or the like, the present invention allows the determination of those wells which contain hybridoma cells which produce antibodies. It is also desirable to utilize the present invention in determining which of the wells of the screening assembly contain hybridoma cells and also contain specific monoclonal antibodies. The present invention allows the handling of all wells, preferably 96, in one step instead of much smaller numbers of wells which are presently being handled by researchers. In addition, in utilizing the beads of the present invention, only an extremely small amount of fluid is withdrawn from the wells upon completion of the individual tests, that is, only the fluid that is retained on the beads is removed from the wells. It is appreciated that the simultaneous transfer of up to 96 protein absorbing substrate units to various other multi-well or multi-tube devices contributes to saving material and labour in the performance of immunoassay tests. The structure of the present invention desirably eliminates or minimizes cross-contamination among the plurality of wells during the simultaneous transfer of the aseptic fluids. The components of the present invention may also be made inexpensively so as to be disposable after use.

The present invention will now be illustrated by way of example with reference to the accompanying drawings, in which, Figures 3 to 10 illustrate embodiments of the invention and Figures 1, 2 and 2a illustrate constructions which are not in accordance with the present invention:

Fig. 1 is a perspective view illustrating a multi-well screening assembly with the tray and carrier plate separated from each other as they may appear before use;

Fig. 2 is a side elevation, partly in section, illustrating the assembled components of the embodiment of Fig. 1;

Fig. 2a is a view similar to Fig 2 illustrating an alternative tray element.

Fig 3 is an enlarged side elevation of the post and bead configuration of the carrier plate, illustrating an alternate construction of the post to that shown in the previous figures, this alternate construction being in accordance with the present invention;

Fig 4 is a perspective view of an alternative carrier plate embodiment of the present invention;

Fig 5 is a side elevation, partly in section, of the carrier plate of Fig 4;

Fig 6 is a side elevation, partly in section, of the carrier plate of Fig 4 assembled onto a tray similar to the tray shown in Fig 1;

Fig 7 is a side elevation, partly in section, of the carrier plate of Fig 4 assembled onto a tray for washing or the like without having individualized wells therein;

Fig 8 is a side elevation, partly in section, illustrating a rack of tubes corresponding in geometry to he carrier plate of Fig 4 shown placed on top of that carrier plate;

Fig. 9 is a side elevation, partly in section, illustrating the assembly of Fig 8 inverted to allow the beads to fall into the tubes; and

Fig 10 is a side elevation, partly in section, illustrating a post and bead deposited inside a test tube.

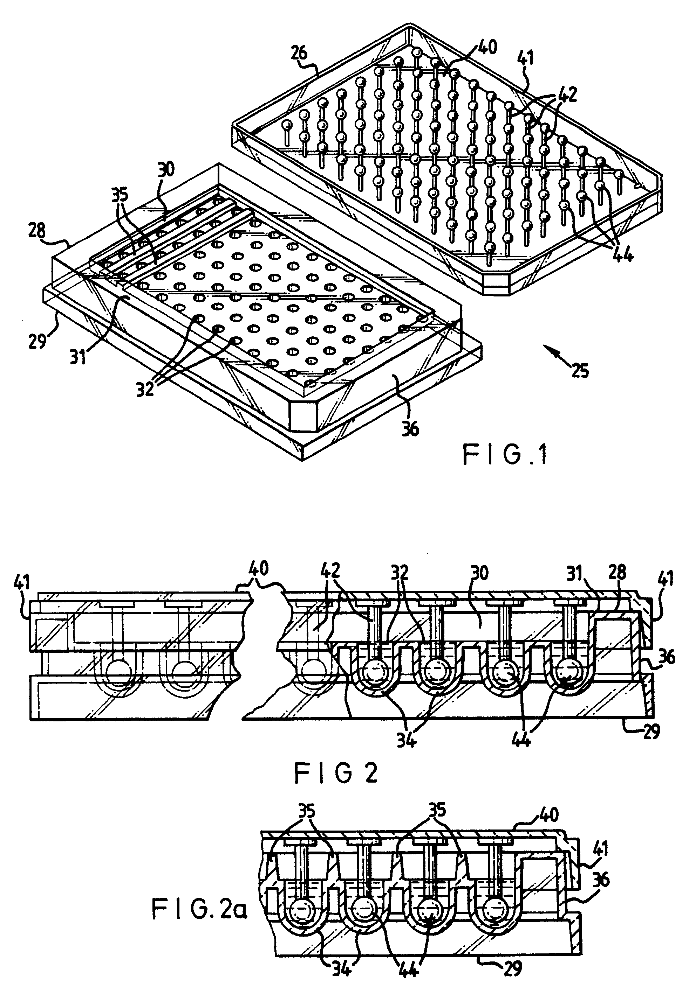

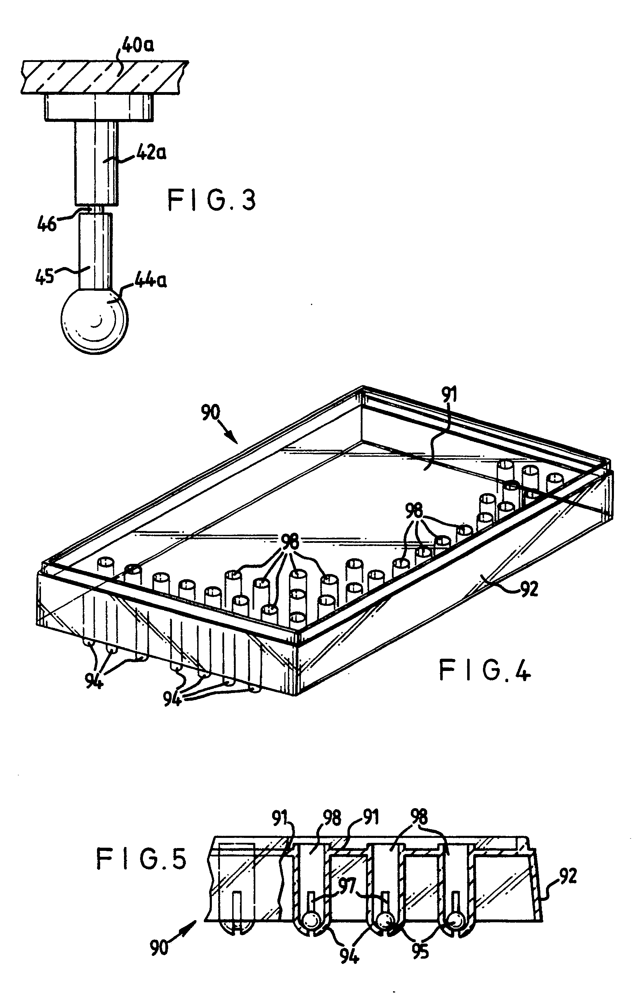

There are shown in Figures 3 to 10 the drawings and will herein be described in detail some preferred, non-limiting embodiments of the invention. Reference is first made to Figures 1, 2 and 2a, to enable comprehension of the succeeding Figures.

In Fig 1 and 2, there is illustrated a multi-well screening assembly 25 which is useful in immunoassay procedures. This assembly is comprised of two general components: a carrier plate 26 and a tray 28 onto which the carrier plate is positioned during use, as more clearly illustrated in Fig 2.

Tray 26 generally has a substantially planar body 29 which may be supported on a flat surface.

Formed within body 29 is a deep recess 30 so that a web 31 surrounds the recess. Formed within recess 30 is a plurality of separated, individualized wells 32, which preferably have cylindrically shaped sidewalls and include a spherically shaped bottom 34 inside the tray body so that fluid may be deposited therein. Wells 32 are preferably formed in the tray in an orthogonal array of rows and columns. In the embodiments being described, there are 96 wells in the tray, with 12 rows each with 8 wells. The individualized wells are provided in the tray to minimize the volume of solution required to coat beads 44. The wells may all be placed in the bottom of one large recess 30 as shown in Fig 2, or each row may be separated with barriers 35 as shown in Fig 2a, or one or two rows only may be separated as seen in Fig 1. The advantage of using a single recess is that it requires only the one-time addition of a fixed volume of solution to submerge all wells. The advantage of separated rows is that the assembly allows for use of individual rows for controls; however, it requires the individual addition of solution to each row. The separation of one or two rows only is a compromise which provides both control provisions and reduced filling operations.

While the volume of each well may vary according to many factors, it is preferred, in the embodiment being described, that each well have the capacity to hold approximately one hundered microlitres of fluid.

In order to minimize expense and render the tray disposable, it is preferred that it be made of plastic material. To minimize the amount of solution used, it is also preferred that the material selected for the tray be made of a non-protein adsorbent material such as polypropylene, polyethylene or the like.

Turning now to carrier plate 26, it is comprised of generally planar support body 40 surrounded by a raised wall 41. The dimensions of wall 41 allow the carrier plate to be positioned on tray 28 so that wall 41 surrounds wall 36 on the tray, as more clearly seen in Fig 2. Protruding from the planar surface of body 40 is a plurality of posts 42. These posts are arranged in the same pattern as wells 32 in the tray and generally correspond to the same number of wells. In the embodiment being described there are thus 96 posts orthogonally arranged in 12 rows of 8 posts each.

When the carrier plate is positioned onto the tray as illustrated in Fig 2, each post depends into one of the wells in the tray. It is preferred that the posts all have the same diameter and length so that consistency and uniformity can be maintained. In the preferred embodiment of the present invention, a substantially spherical bead 44 is connected to the distal end of each post so that the bead depends deepest into the well when the carrier plate is positioned on the tray. In order to allow the beads to enter the wells, it is preferred that they have a diameter of about 3.2 millimeters, although this size may vary according to circumstances.

Carrier plate 26 is preferably an integrally formed or molded structure. Plastics are the materials of choice, such as styrene acrylonitrile, polypropylene, polyethylene, polystyrene and the like. However, irrespective of the material of carrier plate 26 in general, beads 44 must be made from materials which will allow proteins to be adsorbed on the surfaces thereof. As used herein, the term proteins includes any immunogenic substance, be it antigen or antibody. The bead material should be capable of establishing a hydrophobic bond with the protein so that the protein can be strongly adsorbed on the surface of the bead material. It has been found that plastic materials satisfy this criteria of adsorbing proteins from a properly prepared fluid medium which contacts the beads. The preferred bead material is styrene acrylonitrile, although other plastic materials may be employed, such as nylon, polystyrene and the like.

In some immunoassay procedures, such as the RIA test, the automated equipment requires that the bead to which the proteins are bound to be placed in individual test tubes. To this end, the present invention provides a mechanism for removing either the bead or the bead and post from the carrier plate so that it may be deposited in a test tube for further immunoassay procedures. Thus, an alternate post and bead embodiment is illustrated in Fig. 3. Post 42a is shown connected to body 40a of the carrier plate (not shown). This post includes a neck portion 46 which has a diameter significantly reduced from the diameter of the main portion of the post. The reduced diameter of neck portion 46 provides a frangible structure so that bead 44a and a portion 45 of post 42a can be readily broken away from the remainder of the post. Removal of the beads from the carrier plate may be done manually or by appropriately designed automated or semi-automated devices, if desirable. By briefly referring to Fig. 10, it can be seen that bead 44a, with post portion 45, is deposited in the bottom of test tube 46 for further immunoassay testing procedures.

Another embodiment of the present invention is illustrated in Figs. 4- 9. As can be seen by referring particularly to Figs. 4 and 5, this aspect of the invention comprises a carrier plate 90. This plate includes a generally planar support body 91 with a downwardly depending wall 92 surrounding body 91. Also depending downwardly from body 91 is a plurality of separated, individualized pockets 94. These pockets are large enough to hold a bead 95 such as described above in a previous embodiment. In this embodiment, the pockets are orthogonally arranged in rows and columns, with there being preferably 96 such pockets. Each pocket includes one or more slots 97 in its distal end which is the end of the pocket to be placed into a well of a tray as hereinafter described. Slot 97 has a transverse dimension smaller than the diameter of bead 95 so that fluid can enter into the pocket, but the bead will be maintained in the bottom of the pocket during use. At the opposite end of each pocket is a larger opening 98, which is large enough to deposit and subsequently remove the bead from the pocket.

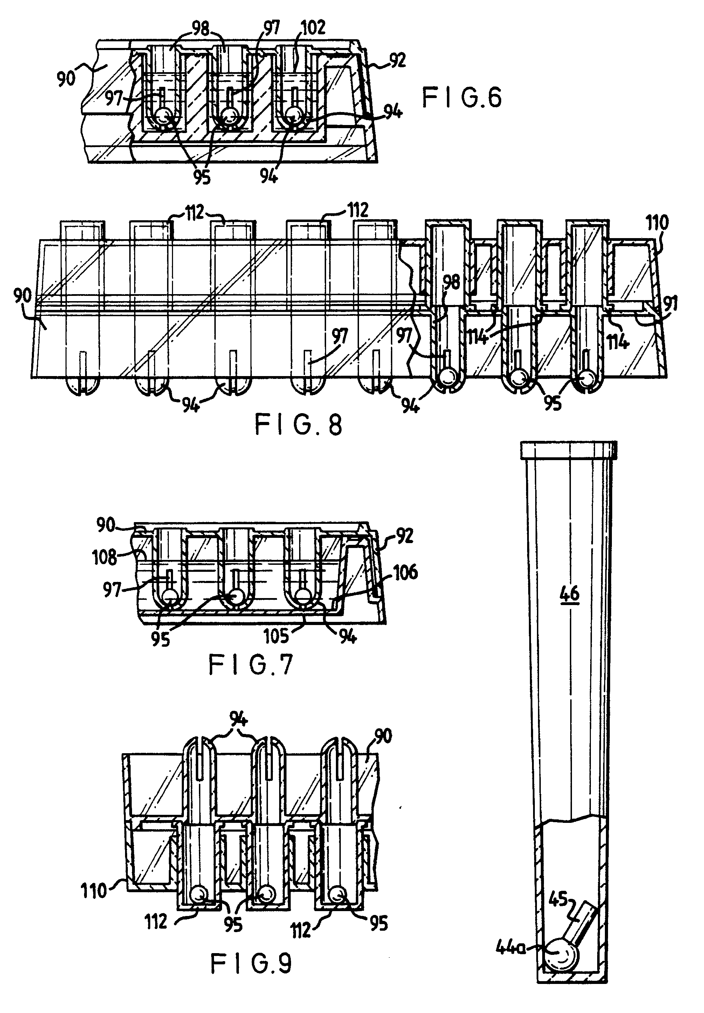

Carrier plate 90 is seen positioned on a tray or plate 100 which is substantially similar to the trays previously described, having separated, individualized wells 101. Each pocket depends into one of the wells containing fluid 102 therein. This fluid enters into the pocket by passing through the slot 97 in each pocket and bathes bead 95 contained in the pocket. Beads 95 are adapted to adsorb proteins on their surfaces and are preferably made of the materials heretofore described. It can be seen in Fig. 6 that the level of fluid 102 can be maintained so that no fluid will exit from the pocket through larger opening 98 therein.

The carrier plate embodiment of Figs. 4 and 5, of course, can be utilized with many different types of fluid carrying plates or trays. One such different tray is illustrated in Fig. 7, wherein tray 105 has one continuous cup-shaped recess 106. Fluid 108 placed in recess 106 will then bathe all of the beads 95 simultaneously. This type of tray 105 may be used for various washings of the beads during the various steps of the immunoassay procedures.

After beads 95 have been treated during the immunoassay procedures, it is desirable to remove them from the carrier plate and place them in individual test tubes for further testing, such as the RIA procedure. To accomplish this, carrier plate 90 is removed from the tray containing the fluid as heretofore described. A rack 110 containing a number of test tubes 112 corresponding in number to pockets 94 is placed over body 91 of carrier plate 90. The open ends 114 of test tubes 112 are aligned over larger openings 98 in the pockets, so that communication is established between the pockets and the test tubes, as more clearly seen in Fig. 8. Once rack 110 is placed onto carrier plate 90 with the test tubes aligned with pockets 94, a quick inversion of the composite assembly will cause beads 95 to drop from the pockets into the test tubes, as illustrated in Fig. 18. Test tubes 112 may then be removed individually to screen in RIA equipment. Fig. 10, as previously mentioned, illustrates a test tube 46, similar to test tube 112, which contains a treated bead therein for further testing purposes.

While the invention as described herein can be used by researchers for the detection and quantification through the immunoassay method, of many different biological substances, it finds significant application in the testing for monoclonal antibodies and, therefore, for specific hybridoma cells. Specifically, a simple test may be performed using the present invention to make a determination in which of the wells there are antibody-producing hybridoma cells. Such a determination is possible due to the interaction between antibody and antigen and the manner of protein treatment of the beads, disks or other bodies which adsorb immunogenic substances thereto. A more complete screening test utilizing the present invention can determine which of the wells that contain hybridoma cells also contain specific monoclonal antibody-producing hybridoma cells.

An example of the aforementioned simple test, to which the present invention, of course, is not limited, is as follows: a carrier plate in accordance with any one of the foregoing embodiments which includes beads, disks or other bodies thereon capable of adsorbing proteins, is positioned on a tray preferably containing 96 wells each containing hybridoma cells covered by HAT (containing Hypoxanthine, Aminopterin, Thymiding) fluid medium. If beads are utilized on the carrier plate, the medium then bathes the beads. This step may last between 15 and 45 minutes and is usually carried out at room temperature and under aseptic conditions. Following this bathing step, the carrier plate, with beads therein or thereon, is removed from the wells containing hybridoma cells, and positioned onto a tray, such as tray 105 illustrated in Fig. 16. This tray may be filled with a selected buffer solution into which the beads are rinsed by immersion. This immersion step may be repeated two or three times. Subsequent to the rinse, the carrier plate with treated beads will be immersed in another tray preferably containing 96 wells, each containing a solution of a chemical agent that will occupy all the sites on the beads that have not adsorbed antibodies from the hybridoma medium as described above. Examples of the chemical agents which may be utilized are ethanol-di-amine or polyvinylpyrrolydone. After this step, which is not mandatory and depends upon the circumstances which may last between 15 and 45 minutes, the beads are again rinsed in the same fashion as previously described. Thereafter, the carrier plate with the treated beads is positioned onto another tray preferably containing 96 wells. If, for example, the immunoassay procedure is an RIA test, each well could contain a medium known as 125I labeled-protein A, or preferably 125-I-labeled antimouse antibodies. After a rinse, the beads may be separated from the carrier plate and transported, by test tubes or the like, to a gamma counter in accordance with known RIA procedures. If, on the other hand, the ELISA procedure is to be utilized, the wells of the tray may be filled with an enzyme-labeled-protein A, or preferably enzyme-labeled anti-mouse antibodies. Following incubation and a rinsing procedure, the reacted beads (with or without separation from the carrier plate) can be placed in a tray which wells are filled with the enzyme substrate linked to a chromogen. After the color reaction develops, this latter tray's wells can be read in a spectrophotometer, or even visually, in accordance with known ELISA procedures. It is understood that other assay procedures may also utilize the multi-well assembly and components of the present invention.

Thus, the present invention provides a multi-well screening device which is useful in immunoassay procedures and which researchers may use in the simultaneous processing of a number of targets in the detection and quantification of biological substances.

## Claims

1. A carrier plate (26), useful in immunoassay procedures and comprising:

    a support body (40a), and

    a plurality of posts (42a), extending from a face of the body (40a) with each post having an enlarged portion (44a) at its distal end adapted to bind an immunogenic substance to its surface, characterized in that each post (42a) has a reduced neck (46) to render it frangible for removal of the enlarged portion from the plate (26).

2. A plate according to claim 1, wherein the enlarged portion is a substantially spherical bead (44a).

3. A plate according claim 1 or 2, wherein the posts are arranged in orthogonal columns and rows.

4. A plate according to any preceding claim, which has 96 posts.

5. A plate according to any preceding claim, wherein the enlarged portions are made of plastics, preferably styrene acrylonitrile.

6. A plate according to claim 5 which is integrally formed or moulded.

7. A plate according to claim 6, wherein the entire plate is made of styrene acrylonitrile.

8. A multi-well screening assembly comprising:

    a tray (28), having a plurality of individualized wells (31), therein; and

    a carrier plate (26), for positioning on the tray and having a plurality of posts (42a), each post extending from the plate to align with a respective one of the wells, the plate being in accordance with any one of the preceding claims.

9. An assembly according to claim 8, wherein each row of wells is separated by a fluid barrier thereby forming a plurality of substantially parallel troughs containing the wells.

10. An assembly according to claim 8, wherein at least one row of wells is separated from the remaining rows in the tray.

11. An assembly according to claim 8, 9 or 10, together with a carrier plate (90) which comprises:

    a generally planar support body (91); and

    a plurality of separate, individualized pockets (94) in the body (91) intended for receipt of individual enlarged portions (44a, 95) after removal from the carrier plate (26) by rupture of the respective frangible neck (46);

    each pocket (94) including an upper opening (98) to allow deposit of the respective removable portion in to the respective pocket and to allow removal of the removable portion from the pocket, and each pocket (94) further including a lower opening (97) which is smaller than the removable portion deposited in the pocket (94) and which allows fluid to enter in to the pocket (94) while the removable portion (95) is in the pocket.

12. A radioimmunoassay ("RIA") for screening of hybridoma cultures for antibody production, characterized in that an assembly according to any of claims 8 to 11 is employed as part of the equipment for the assay, the removable bodies (94) being contacted with the hybridoma cultures while attached to the respective posts (42a) and then being ruptured for removal from the post.

## Revendications

1. Une plaque de support (26), utile dans les modes opératoires des analyses immunologiques et comprenant

    un corps de support 40(a) et
    une pluralité de montants s'étendant au départ d'une face du corps 40(a), chaque montant présentant à son extrémité distale une partie élargie (44a) adaptée de manière à fixer sur sa surface une substance immunogène, caractérisée en ce que chaque montant (42a) présente un col de dimension réduite (46) de manière à le rendre fragile pour l'enlèvement de la partie élargie de la plaque (26).

2. Une plaque suivant la revendication 1, dans laquelle la partie élargie est une perle sensiblement sphérique (44a).

3. Une plaque suivant la revendication 1 ou 2, dans laquelle les montants sont disposés en colonnes et en rangées orthogonales.

4. Une plaque suivant l'une quelconque des revendications précédentes, laquelle plaque a 96 puits.

5. Une plaque suivant l'une quelconque des revendications précédentes, dans laquelle les parties élargies sont fabriquées en matière plastique, de préférence du styrène-acrylonitrile.

6. Une plaque suivant la revendication 5 qui est formée d'une seule pièce ou moulée.

7. Une plaque suivant la revendication 6, dans laquelle l'entièreté de la plaque est fabriquée en styrène-acrylonitrile.

8. Un assemblage pour le criblage en puits multiples comprenant:

un plateau (28) ayant dans sa structure une pluralité de puits individualisés et

une plaque de support (26) destinée à être positionnée sur le plateau et ayant une pluralité de montants (42a), chaque montant s,étendant au départ de la plaque pour s'aligner avec un puits correspondant, la plaque étant en conformité avec l'une quelconque des revendications précédentes.

9. Un assemblage suivant la revendication 8, dans lequel chaque rangée de puits est séparée par une barrière pour la fluide, formant ainsi une pluralité de traverse, sensiblement parallèles contenant les puits.

10. Un assemblage suivant la revendication 8, dans lequel une rangée de puits est séparée des autres rangées dans le plateau.

11. Un assemblage suivant la revendication 8, 9 ou 10, ensemble avec une plaque de support (90) qui comprend:

un corps de support généralement plan (91) et

une pluralité de poches séparées, individualisées (94) dans le corps (91) destinées à recevoir les parties élargies individuelles (44a, 95) après enlèvement de la plaque de support (26) par rupture du col fragile correspondant (46);

chaque poche (94) comprenant un ouverture (98) à sa partie supérieure pour permettre le dépôt de la partie détachable correspondante dans la poche correspondante et pour permettre l'enlèvement de la partie détachable de la poche et chaque poche (94) comprenant en outre, à sa partie inférieure, une ouverture (97) qui est plus petite que la partie détachable déposée dans la poche (94) et qui permet au fluide d'entrer dans la poche (94) alors que la partie détachable (95) est dans la poche.

12. Un radioimmunoessai ( "RIA" ) pour le criblage de cultures d'hybridomes au point de vue de la production d'anticorps, caractérisé en ce qu'on emploie un assemblage suivant l'une quelconque des revendications 8 à 11 comme partie de l'équipement pour l'essai, les corps détachables (94) étant en contact avec les cultures d'hybridomes lorsqu'ils sont attachés aux montants correspondants (42a) et étant ensuite rompus pour l'enlèvement du montant.

**Patentansprüche**

1. Trägerplatte (26), brauchbar bei Immunoassayverfahren umfassend:
einen Trägerkörper (40a) und
eine Vielzahl von Ständern (42a), die sich von einer Fläche des Körpers (40a) fort erstrecken, wobei jeder Ständer einen vergrößerten Teil (44a) an seinem distalen Ende einer Ausbildung hat, durch die eine immunogene Substanz an seiner Oberfläche gebunden wird, dadurch **gekennzeichnet,** daß jeder Ständer (42a) einen reduzierten Hals (46), um diesen brechbar für die Entfernung des vergrößerten Teils von der Platte (26) zu machen, hat.

2. Platte nach Anspruch 1, wobei der vergrößerte Teil eine im wesentlichen spärische Perle (44a) ist.

3. Platte nach Anspruch 1 oder 2, wobei die Pfosten in orthogonalen Säulen und Reihen angeordnet sind.

4. Platte nach einem der vorhergehenden Ansprüche, die 96 Pfosten hat.

5. Platte nach einem der vorhergehenden Ansprüche, wobei die vergrößerten Teile aus Kunststoff, vorzugsweise Styrol-Acrylnitril gemacht sind.

6. Platte nach Anspruch 5, die integral geformt oder preßgeformt ist.

7. Platte nach Anspruch 6, wobei die gesamte Platte aus Styrol-Acrylnitril gemacht ist.

8. Mehr-Schachtsiebanordnung umfassen
ein Tablett (28) mit einer Vielzahl individualisierter Schächte (31) hierin; und
eine Trägerplatte (26) zur Positionierung auf dem Tablett, und mit einer Vielzahl von Ständern (42a), wobei jeder Ständer von der Platte absteht, um bezüglich je eines der Schächte ausgerichtet zu sein, wobei die Platte gemäß einem der vorhergehenden Ansprüche ist.

9. Anordnung nach Anspruch 8, wobei jede Reihe von Schächten durch eine Fluidsperre abgetrennt ist, wodurch eine Vielzahl von im wesentlichen parallelen die Schächte enthaltenden Rinnen gebildet wird.

10. Anordnung nach Anspruch 8, wobei wenigstens eine Reihe von Schächten Von den verbleibenden Reihen in dem Tablett getrennt ist.

11. Anordnung nach Anspruch 8, 9 oder 10 zusammen mit einer Trägerplatte (90), die umfaßt:
einen allgemein planaren Trägerkörper (91); und
eine Vielzahl von gesonderten individualisierten Taschen (94) im Körper (91) zur Aufnahme individueller vergrößerter Teile (44a, 95) nach Entfernung von der Trägerplatte (26) durch Brechen des jeweiligen brechbaren Halses (46);
wobei jede Tasche (94) eine obere Öffnung (98) umfaßt, um die Ablage des jeweils entfernbaren Teils in die jeweilige Tasche und die Entfernung des entfernbaren Teils aus der Tasche zu ermöglichen und wobei jede Tasche (94) eine untere Öffnung (97) umfaßt, die kleiner als der entfernbare in der Tasche (94) abgelegte Teil ist und die es dem Fluid ermöglicht, in die Taschen (94) einzudringen, während der entfernbare Teil (95) sich in der Tasche befindet.

12. Radioimmunoassay ("RIA") zum Absieben von Hybridoma-Kulturen zur Antikörperproduktion, dadurch **gekennzeichnet,** daß eine Anordnung gemäß einem der Ansprüche 8 bis 10 als Teil der Ausrüstung für den Assay verwendet wird, wobei die entfernbaren Körper (94) mit den Hybridoma-Kulturen kontaktiert werden, während sie an den jeweiligen Ständern (42a) befestigt sind und dann zur Entfernung von dem Pfosten gebrochen werden.

FIG.1

FIG 2

FIG.2a

FIG. 3

FIG. 4

FIG. 5

FIG.6

FIG.8

FIG.7

FIG.9